# EUROPÄISCHE PATENTANMELDUNG

(11) **EP 4 335 351 A1**
(43) Veröffentlichungstag der Anmeldung: **13.03.2024**
(21) Anmeldenummer: 23191065.4
(22) Anmeldetag: 11.08.2023
(51) Int. Cl.: A61B 1/00, A61B 1/267, G02F 1/13

(54) **VIDEOLARYNGOSKOP**

(30) Priorität: 08.09.2022 DE 102022122812
(71) Anmelder: Heine Optotechnik GmbH & Co KG, 82205 Gilching (DE)
(72) Erfinder: Entsfellner, Konrad, 82205 Gilching (DE); Michel, Felix, 82205 Gilching (DE)
(74) Vertreter: Flach Bauer & Partner Patentanwälte mbB

(57) **Zusammenfassung**

Ein Videolaryngoskop (12) hat eine Wiedergabeeinheit (16), einen Kameraarm (20) zum Einführen in einen Patienten und eine Kamera (26), die am Kameraarm (20) angeordnet ist. Die Wiedergabeeinheit (16) hat einen Bildschirm (34) mit einem Display (60), einer Hintergrundbeleuchtung (64) sowie einer transflektiven Lage (62), die zwischen dem Display (60) und der Hintergrundbeleuchtung (64) angeordnet ist.

## Beschreibung

Die Erfindung betrifft ein Videolaryngoskop.

Videolaryngoskope sind bekannt und weisen eine an einem Kameraarm angebrachte Kamera auf, die zusammen mit dem Kameraarm in den Patienten eingeführt wird. An einem Bildschirm des Videolaryngoskops wird das Bild der Kamera wiedergegeben, sodass der Eingriff videogeführt sein kann. Insbesondere werden Laryngoskope zur Intubation von Patienten verwendet. Intubationen werden jedoch nicht nur in kontrollierten Umgebungen - wie einem Operationssaal - durchgeführt, sondern erfolgen auch im Rahmen von Notfallbehandlungen am Ort des Unfalls, d. h. häufig im Freien.

Es ist daher Aufgabe der Erfindung, ein Videolaryngoskop bereitzustellen, das zuverlässig auch bei Einsätzen im Freien ohne Hilfsmaßnahmen oder Qualitätsverlusten einsatzfähig ist.

Die Aufgabe wird gelöst durch ein Videolaryngoskop mit einer Wiedergabeeinheit, einem Kameraarm zum Einführen in einen Patienten und einer Kamera, die am Kameraarm angeordnet ist. Die Wiedergabeeinheit weist einen Bildschirm mit einem Display, einer Hintergrundbeleuchtung sowie einer transflektiven Lage auf, die zwischen dem Display und der Hintergrundbeleuchtung angeordnet ist.

Der Erfindung liegt die Erkenntnis zugrunde, dass bei Notfalleinsätzen im Freien die Lichtverhältnisse im Gegensatz zu Innenräumen stark variieren und insbesondere direkte Sonneneinstrahlung die Verwendung von Videolaryngoskopen erschwert. Die Erfinder haben erkannt, dass eine transflektive Lage zwischen Display und Hintergrundbeleuchtung die Lesbarkeit des Displays auch unter direkter Sonneneinstrahlung drastisch erhöht, ohne dabei die für einen medizinischen Eingriff notwendige hohe Videoqualität zu beeinträchtigen. Durch die transflektive Lage ist somit das Display auch bei direkter Sonneneinstrahlung gut ablesbar, wodurch das erfindungsgemäße Videolaryngoskop auch im Freien ohne Qualitätsverluste oder Hilfsmaßnahmen einsatzfähig ist.

Der Kameraarm und die Wiedergabeeinheit können als separate Bauteile ausgeführt sein, die miteinander verbunden sind, insbesondere lösbar. Alternativ können die Wiedergabeeinheit und der Kameraarm zusammen einstückig, also als ein Gerät ausgebildet sein.

Denkbar ist auch, dass der Kameraarm zum Einführen in einen separaten Spatel ausgebildet ist. Alternativ kann der Kameraarm als Spatel ausgebildet sein.

Das Display ist insbesondere auf der Vorderseite des Bildschirms angeordnet, d.h. dem Benutzer des Videolaryngoskops zugewandt.

Vorzugsweise ist die Wiedergabeeinheit mit der Kamera verbunden und dazu eingerichtet, das von der Kamera aufgenommene Bild wiederzugeben, insbesondere in Echtzeit.

Für eine besonders gute Lesbarkeit kann die transflektive Lage einen Reflektionsgrad von 0,1 bis 0,8, insbesondere von 0,1 bis 0,5 für Umgebungslicht aufweisen und/oder einen Transmissionsgrad von 0,9 bis 0,2, insbesondere 0,9 bis 0,5 für Licht aufweisen, das von der Hintergrundbeleuchtung emittierten ist.

In einer Ausführungsform ist das Display ein Vollfarbdisplay und/oder hat eine Auflösung von wenigstens 240x180 Pixel, insbesondere wenigstens 320x240 Pixel, besonders bevorzugt wenigstens 800x480 Pixel, wodurch eine optimale Wiedergabe für medizinische Zwecke gewährleistet ist.

Unter einem Vollbfarbdisplay wird im Rahmen dieser Offenbarung ein Display mit wenigstens 16 Bit Farben, insbesondere mit wenigstens 24 Bit Farben verstanden.

Beispielsweise ist das Display ein TFT-Display ist, insbesondere ein TFT-LCD, wodurch die Qualität der Wiedergabe weiter verbessert wird.

In einer Ausgestaltung weist die Wiedergabeeinheit ein Gehäuse mit einer Bildschirmöffnung auf, wobei der Bildschirm im Gehäuse, insbesondere in der Bildschirmöffnung, angeordnet ist, wodurch das Videolaryngoskop besonders robust ist.

Beispielsweise weist das Gehäuse einen Rahmen auf, insbesondere aus Metall, in dem die Bildschirmöffnung ausgebildet ist. Der Rahmen kann aus Aluminium sein und führt zu einer besonders stabilen Wiedergabeeinheit.

In einer Ausführungsform weist das Gehäuse eine Abdeckung, insbesondere aus Kunststoff auf, die am Rahmen befestigt ist, insbesondere wobei die Abdeckung mittels einer die Bildschirmöffnung umlaufenden Dichtung am Rahmen befestigt ist. Dadurch wird das Gehäuse zuverlässig und kostengünstig verschlossen.

Zum Schutz des Bildschirms kann die Wiedergabeeinheit ein Deckglas aufweisen, das die Bildschirmöffnung verschließt.

In einer Ausführungsform ist der Bildschirm, insbesondere das Display, an das Deckglas angeklebt, insbesondere durch eine Klebstoffschicht, die die dem Deckglas zugewandte Oberfläche des Bildschirms vollflächig bedeckt. Auf diese Weise wird eine besonders hohe Wiedergabequalität erreicht. Insbesondere führt das vollflächige Verkleben dazu, dass störende Lichtreflexe, zum Beispiel durch Sonneneinstrahlung, reduziert werden, da kein Luftspalt zwischen dem Deckglas und dem Bildschirm vorhanden ist.

Zur Herstellung der Klebstoffschicht kann ein Gelkleber eingesetzt werden.

Beispielsweise wird der Bildschirm vom Deckglas getragen und/oder das Deckglas und der Bildschirm bilden eine Baugruppe.

Im Rahmen dieser Offenbarung bedeutet "vollflächig bedeckt" zum Beispiel, dass zumindest die aktive Anzeigefläche des Bildschirms, insbesondere des Displays, bedeckt ist.

Für eine äußerst belastbare Befestigung kann das Gehäuse, insbesondere der Rahmen, eine Stufe aufweisen, die von der Bildschirmöffnung ausgeht und die die Bildschirmöffnung umläuft, wobei das Deckglas auf der Stufe aufliegt, insbesondere wobei ein Abstand zwischen der Vorderseite des Rahmens und der Stufe größer ist als die Dicke des Deckglases.

Beispielsweise ist Stufe von der Vorderseite ausgehend ausgebildet, d.h. von der der Abdeckung abgewandten Seite des Rahmens.

Das Gehäuse steht insbesondere über das Deckglas hervor.

In einer Ausgestaltung ist das Deckglas auf der Stufe mittels Klebstoff befestigt, wobei der Klebstoff die Bildschirmöffnung umläuft und/oder wobei der Klebstoff in einem radialen Spalt zwischen Deckglas und Gehäuse vorgesehen ist, insbesondere ausschließlich in dem Spalt vorgesehen ist. Auf diese Weise wird das Gehäuse zuverlässig und langlebig dicht verschlossen. Zum Beispiel umläuft der Klebstoff die Bildschirmöffnung vollständig und/oder es ist keine Luft zwischen dem Rahmen und dem Deckglas im Spalt vorhanden.

Zur weiteren Verbesserung der Langlebigkeit kann die Wiedergabeeinheit eine Steuerungselektronik für den Bildschirm aufweisen, insbesondere wobei die Steuerungselektronik innerhalb des Gehäuses angeordnet ist.

In einer Ausführungsform weist das Videolaryngoskop einen Griff auf, wobei der Kameraarm an einem ersten Ende des Griffs am Griff befestigt ist und/oder wobei die Wiedergabeeinheit an einem zweiten Ende des Griffs am Griff befestigt ist, insbesondere lösbar.

Das erste Ende und das zweite Ende des Griffs sind insbesondere zueinander entgegengesetzt.

Beispielweise geht der Griff in den Kameraarm über und/oder der Griff und der Kameraarm sind zusammen einstückig ausgeführt.

Die Wiedergabeeinheit ist am Griff insbesondere werkzeugfrei lösbar befestigt, beispielsweise mittels eines Rastmechanismus.

Denkbar ist jedoch auch, dass die Wiedergabeeinheit permanent am Griff befestigt ist bzw. die Wiedergabeeinheit und der Griff zusammen einstückig ausgeführt sind.

In einer Ausführungsform weist der Griff eine Aufnahme auf und die Wiedergabeeinheit weist einen Eingriffsabschnitt auf, der in der Aufnahme aufgenommen ist. Auf diese Weise wird ein modulares Videolaryngoskop bereitgestellt, bei dem die Wiedergabeeinheit vom übrigen Videolaryngoskop getrennt werden kann. Dadurch können die verschiedenen Teile getrennt voneinander wiederaufbereitet werden, wodurch für den Griff und/oder den Kameraarm aggressivere Wiederaufbereitungsmethoden verwendet werden können. Dieser Aspekt ist unabhängig von der Verwendung der transflektiven Lage.

Es ist dadurch auch denkbar, dass verschiedene Griffe und Kameraarme mit der gleichen Wiedergabeeinheit verwendet werden können, zum Beispiel Kameraarme mit verschiedenen Krümmungen, ohne Krümmung und/oder mit verschiedenen Längen für verschiedene Spatel (Macintosh-Spatel, Miller-Spatel, hyperangulierter Spatel, Kinderspatel, etc.). Auf diese Weise kann die Wiedergabeeinheit für verschiedene Einsatzzwecke verwendet werden, wodurch Systemkosten beim Endanwender eingespart werden.

Die Aufnahme kann zylindrisch oder konisch sein.

In einem Aspekt hat die Aufnahme eine Wandung, von der eine Rippe nach innen vorsteht und der Eingriffsabschnitt hat wenigstens ein elastisches Rastelement, insbesondere wenigstens eine Rastkugel am Außenumfang, das hinter der Rippe verrastet ist, wodurch sich die Wiedergabeeinheit zuverlässig und intuitiv am Griff befestigen lässt.

Dabei ist "hinter" dahingehend zu verstehen, dass das Rastelement im vollständig eingeführten Zustand auf der vom zweiten Ende des Griffes abgewandten Seite der Rippe angeordnet ist.

Weitere Merkmale und Vorteile der Erfindung ergeben sich aus der nachfolgenden Beschreibung sowie aus den beigefügten Zeichnungen, auf die Bezug genommen wird. In den Zeichnungen zeigen:
- Fig. 1: ein Videolaryngoskopsystem mit einem Videolaryngoskop gemäß einer Ausführungsform der Erfindung in Explosionsansicht,
- Fig. 2: eine Schnittansicht durch eine Aufnahme eines Griffs mit eingesetztem Eingriffsabschnitt einer Wiedergabeeinheit des Videolaryngoskops gemäß Figur 1 schematisch,
- Fig. 3: eine schematische Schnittansicht durch eine Wiedergabeeinheit des Videolaryngoskops gemäß Figur 1, und
- Fig. 4: einen schematischen Schnitt durch einen Bildschirm der Wiedergabeeinheit gemäß Figur 3.

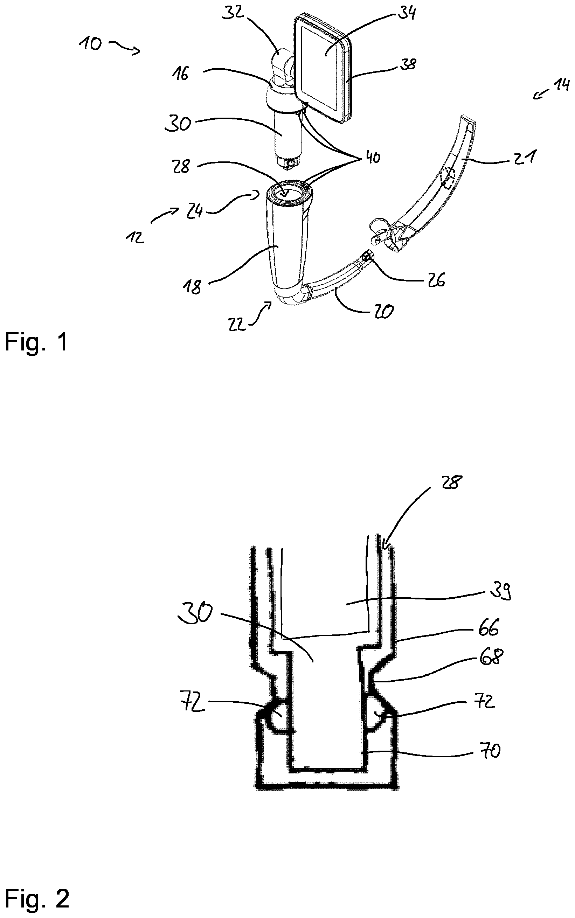

In Figur 1 ist ein Videolaryngoskopsystem 10 mit einem Videolaryngoskop 12 gemäß einer Ausführungsform der Erfindung und einer Aufsatzbaugruppe 14 in Explosionsansicht dargestellt.

Das Videolaryngoskop 10 umfasst eine Wiedergabeeinheit 16, einen Griff 18 sowie einen Kameraarm 20.

Die Aufsatzbaugruppe 14 umfasst im einfachsten Fall lediglich einen Spatel 21, der auf den Kameraarm 20 aufschiebbar ist. Denkbar ist auch, dass im Spatel 21 eine Schutzkappe (in Figur 1 gestrichelt angedeutet) der Aufsatzbaugruppe 14 vorgesehen ist. Die Schutzkappe wird beim Aufschieben des Spatels 21 auf den Kameraarm 20 vom Kameraarm 20 mitgenommen und somit am Kameraarm 20 appliziert.

Der Griff 18 des Videolaryngoskops 10 weist ein erstes Ende 22 und ein zweites Ende 24 auf, die einander entgegengesetzt sind.

Am ersten Ende 22 ist der Kameraarm 20 am Griff 18 befestigt. Im gezeigten Ausführungsbeispiel geht der Griff 18 in den Kameraarm 20 über. Der Griff 18 und der Kameraarm 20 können daher einstückig, also als ein Teil ausgebildet sein.

Am Kameraarm 20 ist eine Kamera 26 des Videolaryngoskops 12 vorgesehen, die insbesondere im Bereich der Spitze angeordnet ist. Das Sichtfeld der Kamera 26 weist vom Griff 18 weg.

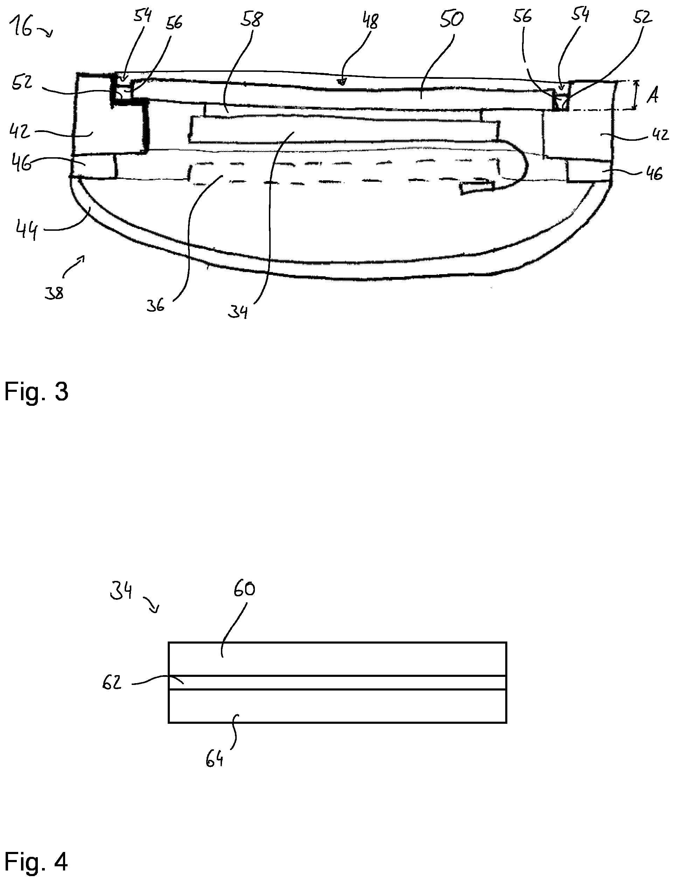

Die Wiedergabeeinheit 16 weist einen Eingriffsabschnitt 30, einen Gelenksabschnitt 32, einen Bildschirm 34, eine Steuerungselektronik 36 (Fig. 3) sowie ein Gehäuse 38 auf.

Die Wiedergabeeinheit 16 ist am zweiten Ende 24 des Griffs 18 am Griff 18 befestigt.

Hierzu weist der Griff 18 im gezeigten Ausführungsbeispiel am zweiten Ende 24 eine Aufnahme 28 auf, in die der Eingriffsabschnitt 30 der Wiedergabeeinheit 16 eingreift.

Die Aufnahme 28 sowie der Eingriffsabschnitt 30 sind derart ausgebildet, dass die Befestigung der Wiedergabeeinheit 16 vom Griff 18 lösbar ist.

In Figur 2 ist ein Schnitt durch die Aufnahme 28 mit eingesetztem Eingriffsabschnitt 30 dargestellt.

Im Eingriffsabschnitt ist beispielsweise eine Stromquelle 39, wie eine Batterie, der Wiedergabeeinheit angeordnet.

Die Aufnahme 28 ist im gezeigten Beispiel zylindrisch. Denkbar ist jedoch auch, dass die Aufnahme 28 konisch ausgebildet ist und sich vom zweiten Ende 24 zum ersten Ende 22 hin verjüngt.

Die Aufnahme 28 hat eine innere Wandung 66, die die Aufnahme 28 radial begrenzt. An der Wandung 66 ist eine Rippe 68 vorgesehen, die sich in Umfangsrichtung erstreckt und beispielsweise in Umfangsrichtung geschlossen ist.

Die Rippe 68 ist im hinteren Drittel der Aufnahme 28 vorgesehen, also in dem zum ersten Ende 22 am nächsten liegenden Drittel der Aufnahme 28 in Bezug zur axialen Richtung.

Der Eingriffsabschnitt 30 ist komplementär zur Aufnahme 28 ausgebildet und im eingeführten Zustand vollständig in der Aufnahme 28 aufgenommen.

Der Eingriffsabschnitt 30 weist eine Außenfläche 70 auf, an der Rastelemente 72 vorgesehen sind. Die Rastelemente 72 sind elastisch nach radial innen bewegbar und radial nach außen vorgespannt.

Im gezeigten Ausführungsbeispiel sind die Rastelemente 72 mehrere Rastkugeln, die hinter der Rippe 68 verrastet sind, d.h. zwischen der Rippe 68 und dem ersten Ende 22, wenn der Eingriffsabschnitt 30 vollständig in die Aufnahme 28 eingeführt ist.

Beim Einsetzen und Entfernen der Wiedergabeeinheit 16 aus dem Griff 18 werden die Rastelemente 72 bzw. Rastkugeln durch die Rippe 68 nach innen bewegt, um den Eingriffsabschnitt 30 in axialer Richtung bewegen zu können.

Damit kann der Griff 18 mitsamt Kameraarm 20 von der Wiedergabeeinheit 16 gelöst werden, um separat gereinigt und desinfiziert zu werden.

Am zweiten Ende 24 des Griffs 18 sowie am Eingriffsabschnitt 30 sind zudem elektrische Kontakte 40 vorgesehen, mittels denen die Kamera 26 mit dem Bildschirm 34 bzw. der Steuerungselektronik 36 elektrisch verbunden ist, wenn die Wiedergabeeinheit 16 im Griff 18 eingesetzt ist.

Am Eingriffsabschnitt 30 ist der Bildschirm 34 mittels des Gelenksabschnitts 32 befestigt. Der Gelenksabschnitt 32 ist dabei an dem vom Griff 18 abgewandten Ende des Eingriffsabschnitts 30 vorgesehen.

Mittels des Gelenksabschnitts 32 ist der Bildschirm 34 gegenüber dem Eingriffsabschnitt 30 und somit gegenüber dem Griff 18 und dem Kameraarm 20 drehbar.

Die Wiedergabeeinheit 16, genauer gesagt der Bildschirm 34, ist dazu eingerichtet, das von der Kamera 26 aufgenommene Bild in Echtzeit wiederzugeben, um einen videogeführten Eingriff, insbesondere eine Intubation, am Patienten vorzunehmen.

In Figur 3 ist ein Schnitt durch das Gehäuse 38, den Bildschirm 34 und die Steuerungselektronik 36 der Wiedergabeeinheit 16 schematisch dargestellt.

Das Gehäuse 38 weist im gezeigten Ausführungsbeispiel einen Rahmen 42, eine Abdeckung 44, eine Dichtung 46 und eine Bildschirmöffnung 48 auf.

Der Rahmen 42 ist aus einem Metall, beispielsweise Aluminium, und umgibt die Bildschirmöffnung 48 vollständig, die im Rahmen 42 ausgebildet ist. Der Rahmen 42 bildet die Vorderseite des Gehäuses 38.

Die Abdeckung 44 ist beispielsweise aus Kunststoff und schalenförmig ausgebildet. Sie ist am Rahmen 42 befestigt, genauer gesagt an der Rückseite des Rahmens 42, also der Seite des Rahmens, die nicht die Vorderseite des Gehäuses 38 darstellt.

Die Abdeckung 44 ist im gezeigten Ausführungsbeispiel mittels der Dichtung 46, beispielsweise aus Gummi, am Rahmen 42 befestigt. Die Dichtung 46 umläuft ebenfalls die Bildschirmöffnung 48 und dichtet somit den Übergang zwischen dem Rahmen 42 und der Abdeckung 44 vollständig ab.

Innerhalb des Gehäuses 38 sind der Bildschirm 34 und die Steuerungselektronik 36 angeordnet.

Dabei ist die Steuerungselektronik 36 hinter dem Bildschirm 34, also zwischen dem Bildschirm 34 und der Abdeckung 44 vorgesehen.

Die Steuerungselektronik 36 und der Bildschirm 34 sind mittels eines flexiblen elektrischen Verbinders, beispielsweise einem Flex-PCB, elektrisch verbunden.

Die Steuerungselektronik 36 ist zur Steuerung des Bildschirms 34 konfiguriert. Die Kamera 26 ist daher über die Steuerungselektronik 36 mit dem Bildschirm 34 verbunden.

Im gezeigten Ausführungsbeispiel ist der Bildschirm 34 sogar innerhalb des Rahmens 42 in der Bildschirmöffnung 48 angeordnet.

Der Bildschirm 34 wird innerhalb des Gehäuses 38 durch ein Deckglas 50 der Wiedergabeeinheit 16 gehalten.

Das Deckglas 50 ist in den Rahmen 42 eingesetzt und deckt die Bildschirmöffnung 48 vollständig ab.

Hierzu weist der Rahmen 42 an seiner Vorderseite eine Senkung auf, die eine Stufe 52 bildet. Die Stufe 52 ist somit von der Vorderseite des Rahmens ausgehend um einen Abstand A nach hinten, d.h. zur Abdeckung 44 hin versetzt.

Die Stufe 52 geht von der Bildschirmöffnung 48 aus und umläuft die Bildschirmöffnung 48 vollständig entlang ihres Umfangs.

Das Deckglas 50 liegt auf der Stufe 52 auf, wodurch die Bildschirmöffnung 48 verschlossen wird.

Hierzu ist das Deckglas 50 größer als die Bildschirmöffnung 48 ausgeführt, d.h. dass die Fläche des Deckglases 50 größer ist als die Fläche der Bildschirmöffnung 48 senkrecht zur Mittelachse der Bildschirmöffnung 48.

Die Dicke des Deckglas 50 ist dabei so gewählt, dass sie kleiner ist als der Abstand A zwischen der Vorderseite des Rahmens 42 und der Stufe 52. Der Rahmen 42 steht somit über das Deckglas 50 hervor, wodurch das Deckglas 50 gut vor Stößen geschützt ist.

In radialer Richtung, d.h. senkrecht zur Mittelachse der Bildschirmöffnung 48, ist die Stufe 52 größer ausgeführt als das Deckglas 50. Somit entsteht ein Spalt 54 radial zwischen den Stirnseiten des Deckglases 50 und dem Rahmen 42, der die Bildschirmöffnung 48 vollständig umläuft.

In den Spalt 54 ist Klebstoff 56 eingebracht, der das Deckglas 50 am Rahmen 42 befestigt, genauer gesagt auf der Stufe 52. Der Klebstoff 56 umläuft dabei die Bildschirmöffnung 48 vollständig, sodass die Bildschirmöffnung 48 vollständig und dicht verschlossen wird.

Der Klebstoff 56 ist insbesondere ausschließlich im Spalt 54 vorgesehen. Beispielsweise füllt der Klebstoff 56 den Spalt 54 vollständig aus, sodass keine Luft zwischen dem Rahmen 42 und dem Deckglas 50 im Spalt 54 vorhanden ist.

Auf der Rückseite des Deckglases 50, d. h. der Oberfläche des Deckglases 50, die der Abdeckung 44 zugewandt ist, ist der Bildschirm 34 am Deckglas 50 befestigt.

Die Befestigung der Vorderseite des Bildschirms 34 an der Rückseite des Deckglases 50 erfolgt mittels einer Klebstoffschicht 58.

Die Klebstoffschicht 58 bedeckt die Vorderseite des Bildschirms 34 d.h. die dem Deckglas 50 zugewandt Oberfläche des Bildschirms 34, vollflächig. Im gezeigten Ausführungsbeispiel ist die aktive Anzeigefläche des Bildschirms 34 komplett von der Klebstoffschicht 58 bedeckt. Als Klebstoffschicht 58 kann ein Gelkleber verwendet werden.

Durch die Klebstoffschicht 58 wird eine permanente Befestigung des Bildschirms 34 am Deckglas 50 erreicht, sodass der Bildschirm 34 und das Deckglas 50 eine Baugruppe bilden können. Insbesondere wird der Bildschirm 34 vollständig vom Deckglas 50 getragen und ist nicht anderweitig im Gehäuse 38 befestigt.

In Figur 4 ist der Bildschirm 34 schematisch im Schnitt dargestellt.

Der Bildschirm 34 weist ein Display 60, eine transflektive Lage 62 sowie eine Hintergrundbeleuchtung 64 auf.

Das Display 60 ist beispielsweise ein Vollfarbdisplay mit wenigstens 16 Bit, insbesondere mit wenigstens 24 Bit Farben. Das Display 60 kann als TFT-Display (Thin-Film-Transistor-Display), insbesondere als TFT-LCD (Thin-Film-Transistor-Liquid-Crystal-Display) ausgebildet sein.

Beispielsweise hat das Display 60 eine Auflösung von wenigstens 240x180 Pixel, insbesondere wenigstens 320x240 Pixel, besonders bevorzugt wenigstens 800x480 Pixel.

Das Display 60 ist auf der Vorderseite des Bildschirms 34 angeordnet und weist somit zur Vorderseite des Gehäuses 38 bzw. der Wiedergabeeinheit 16. Insbesondere ist es das Display 60, das mit der Klebstoffschicht 58 versehen und an der Rückseite des Deckglases 50 befestigt ist.

Die Hintergrundbeleuchtung 64 ist auf der Rückseite des Displays 60 angeordnet und weist beispielsweise LEDs als Lichtquellen auf.

Die transflektive Lage 62 ist zwischen dem Display 60 und der Hintergrundbeleuchtung 64 angeordnet.

Die transflektive Lage 62 weist eine Größe in radialer Richtung auf, die zumindest der aktiven Anzeigefläche des Displays 60 und/oder der aktiven Fläche der Hintergrundbeleuchtung 64 entspricht. Die transflektive Lage 62 ist derart angeordnet, dass sie zumindest die aktive Anzeigefläche des Display 60 vollständig auf der Rückseite des Displays 60 bedeckt.

Die transflektive Lage 62 hat einen Reflektionsgrad von 0,1 bis 0,8, insbesondere 0,1 bis 0,5 für Umgebungslicht, also für Licht, das von vorne, d.h. durch das Display 60 auf die transflektive Lage 62 trifft.

Für Licht, das von hinten auf die transflektive Lage 62 trifft, d.h. für das von der Hintergrundbeleuchtung 64 emittiert Licht, hat die transflektive Lage 62 einen Transmissionsgrad von 0,9 bis 0,2, insbesondere von 0,9 bis 0,5.

Durch die transflektive Lage 62 wird erreicht, dass der Bildschirm 34 auch bei ungünstigen Beleuchtungsverhältnissen lesbar bleibt, insbesondere im Freien und bei äußerst hellen Umgebungslichtverhältnissen. Ein videogeführter Eingriff an Patienten ist somit ohne Probleme auch im Freien bei direkter Sonneneinstrahlung möglich, insbesondere ohne Hilfsmittel, wie einer Verdunkelung. Durch die vollflächige Klebstoffschicht 58 wird zudem die Anzeigequalität des Bildschirms 34 weiter verbessert.

Auf diese Weise wird somit ein Videolaryngoskop 12 bereitgestellt das sowohl äußerst robust, insbesondere wasserdicht, als auch im Freien zuverlässig einsatzfähig ist.

Die Erfindung wurde beispielhaft anhand eines zweistückigen Videolaryngoskops 12 aus der Wiedergabeeinheit 16 als ein Teil sowie dem Griff 18 und dem Kameraarm 20 als das zweite Teil.

Denkbar ist jedoch ebenfalls, dass das Videolaryngoskop 12 einstückig ist, d.h. die Wiedergabeeinheit 16 permanent am Griff 18 bzw. dem Kameraarm 20 befestigt ist.

Zudem wurde beispielhaft ein Videolaryngoskopsystem 10 mit einem zum Videolaryngoskop 12 separaten Spatel 21 beschrieben. Es ist jedoch ebenso denkbar und erfindungsgemäß, dass der Kameraarm 20 bereits selbst derart ausgebildet ist, insbesondere durch seine Form, dass er den Spatel bildet. In diesem Fall ist kein separater Spatel 21 notwendig.

Ebenfalls ist es denkbar, dass die Wiedergabeeinheit 16 mit verschiedenen Griffen 18 und Kameraarmen 20 verwendet werden kann. Beispielweise weisen die Griffe 18 jeweils die gleiche Aufnahme 28 auf, jedoch können die Kameraarme 20 unterschiedlich, in Abhängigkeit der benötigten Funktion. Zum Beispiel können verschiedene Kameraarme 20 mit verschiedenen Krümmungen, ohne Krümmung und/oder mit verschiedenen Längen zum Einsatz kommen, auf denen jeweils ein spezieller Spatel (Macintosh-Spatel, Miller-Spatel, hyperangulierter Spatel, Kinderspatel, etc.) aufgesetzt werden kann. Dieser Aspekt ist unabhängig von der Verwendung der transflektiven Lage.

## Patentansprüche

1. Videolaryngoskop mit einer Wiedergabeeinheit (16), einem Kameraarm (20) zum Einführen in einen Patienten und einer Kamera (26), die am Kameraarm (20) angeordnet ist,
wobei die Wiedergabeeinheit (16) einen Bildschirm (34) mit einem Display (60), einer Hintergrundbeleuchtung (64) sowie einer transflektiven Lage (62) aufweist, die zwischen dem Display (60) und der Hintergrundbeleuchtung (64) angeordnet ist.

2. Videolaryngoskop nach Anspruch 1, **dadurch gekennzeichnet, dass** die transflektive Lage (62) einen Reflektionsgrad von 0,1 bis 0,8, insbesondere von 0,1 bis 0,5, für Umgebungslicht und/oder einen Transmissionsgrad von 0,9 bis 0,2, insbesondere von 0,9 bis 0,5 für Licht aufweist, das von der Hintergrundbeleuchtung (64) emittierten ist.

3. Videolaryngoskop nach Anspruch 1 oder 2, **dadurch gekennzeichnet, dass** das Display (60) ein Vollfarbdisplay ist und/oder eine Auflösung von wenigstens 240x180 Pixel, insbesondere wenigstens 320x240 Pixel, besonders bevorzugt wenigstens 800x480 Pixel hat.

4. Videolaryngoskop nach einem der vorhergehenden Ansprüche, **dadurch gekennzeichnet, dass** das Display (60) ein TFT-Display ist, insbesondere ein TFT-LCD.

5. Videolaryngoskop nach einem der vorhergehenden Ansprüche, **dadurch gekennzeichnet, dass** die Wiedergabeeinheit (16) ein Gehäuse (38) mit einer Bildschirmöffnung (48) aufweist, wobei der Bildschirm (34) im Gehäuse (38), insbesondere in der Bildschirmöffnung (48) angeordnet ist.

6. Videolaryngoskop nach Anspruch 5, **dadurch gekennzeichnet, dass** das Gehäuse (38) einen Rahmen (42) aufweist, insbesondere aus Metall, in dem die Bildschirmöffnung (48) ausgebildet ist.

7. Videolaryngoskop nach Anspruch 6, **dadurch gekennzeichnet, dass** das Gehäuse (38) eine Abdeckung (44), insbesondere aus Kunststoff aufweist, die am Rahmen (42) befestigt ist, insbesondere wobei die Abdeckung (44) mittels einer die Bildschirmöffnung (48) umlaufenden Dichtung am Rahmen (42) befestigt ist.

8. Videolaryngoskop nach Anspruch 5 oder 6, **dadurch gekennzeichnet, dass** die Wiedergabeeinheit (16) ein Deckglas (50) aufweist, das die Bildschirmöffnung (48) verschließt.

9. Videolaryngoskop nach Anspruch 8, **dadurch gekennzeichnet, dass** der Bildschirm (34), insbesondere das Display (60), an das Deckglas (50) angeklebt ist, insbesondere durch eine Klebstoffschicht (58), die die dem Deckglas (50) zugewandte Oberfläche des Bildschirms (34) vollflächig bedeckt.

10. Videolaryngoskop nach Anspruch 8 oder 9, **dadurch gekennzeichnet, dass** das Gehäuse (38), insbesondere der Rahmen (42), eine Stufe (52) aufweist, die von der Bildschirmöffnung (48) ausgeht und die die Bildschirmöffnung (48) umläuft, wobei das Deckglas (50) auf der Stufe (52) aufliegt, insbesondere wobei ein Abstand zwischen der Vorderseite des Rahmens (42) und der Stufe (52) größer ist als die Dicke des Deckglases (50).

11. Videolaryngoskop nach Anspruch 10, **dadurch gekennzeichnet, dass** das Deckglas (50) auf der Stufe (52) mittels Klebstoff (56) befestigt ist, wobei der Klebstoff (56) die Bildschirmöffnung (48) umläuft und/oder wobei der Klebstoff (56) in einem radialen Spalt (54) zwischen Deckglas (50) und Gehäuse (38) vorgesehen ist, insbesondere ausschließlich in dem Spalt (54) vorgesehen ist.

12. Videolaryngoskop nach einem der vorhergehenden Ansprüche, **dadurch gekennzeichnet, dass** die Wiedergabeeinheit (16) eine Steuerungselektronik (36) für den Bildschirm (34) aufweist, insbesondere wobei die Steuerungselektronik (36) innerhalb des Gehäuses (38) angeordnet ist.

13. Videolaryngoskop nach einem der vorhergehenden Ansprüche, **dadurch gekennzeichnet, dass** das Videolaryngoskop (12) einen Griff (18) aufweist, wobei der Kameraarm (20) an einem ersten Ende (22) des Griffs (18) am Griff (18) befestigt ist und/oder wobei die Wiedergabeeinheit (16) an einem zweiten Ende (24) des Griffs (18) am Griff (18) befestigt ist, insbesondere lösbar.

14. Videolaryngoskop nach Anspruch 13, **dadurch gekennzeichnet, dass** der Griff (18) eine Aufnahme (28) aufweist und die Wiedergabeeinheit (16) einen Eingriffsabschnitt (30) aufweist, der in der Aufnahme (28) aufgenommen ist, insbesondere wobei die Aufnahme (28) eine Wandung (66) aufweist, von der eine Rippe (68) nach innen vorsteht und wobei der Eingriffsabschnitt (30) wenigstens ein elastisches Rastelement (72), insbesondere wenigstens eine Rastkugel an seiner Außenfläche (70) aufweist, das hinter der Rippe (68) verrastet ist.
